(19) Europäisches Patentamt
European Patent Office
Office européen des brevets

(11) Veröffentlichungsnummer: **0 116 169**
**A2**

(12) **EUROPÄISCHE PATENTANMELDUNG**

(21) Anmeldenummer: **83113090.1**

(22) Anmeldetag: **24.12.83**

(51) Int. Cl.³: **C 07 C 49/92**
C 07 C 45/77, C 03 C 17/28
G 01 N 24/00

(30) Priorität: **15.01.83 DE 3301225**

(43) Veröffentlichungstag der Anmeldung:
**22.08.84 Patentblatt 84/34**

(84) Benannte Vertragsstaaten:
**CH DE FR GB IT LI NL SE**

(71) Anmelder: **Merck Patent Gesellschaft mit beschränkter Haftung**
**Frankfurter Strasse 250**
**D-6100 Darmstadt(DE)**

(72) Erfinder: **Rau, Axel, Dr.**
**Hans-Thoma-Strasse 8**
**D-7798 Pfullendorf(DE)**

(72) Erfinder: **Franz, Klaus-Dieter, Dr.**
**Insterburgstrasse 12**
**D-6233 Kelkheim(DE)**

(54) **Verfahren zur Herstellung wasserfreier, fluorhaltiger 1,3-Diketonato-Metall-Komplexe.**

(57) Durch ein neues, allgemein anwendbares Verfahren können durch Umsetzung von wasserfreien Metallderivaten flüchtiger, metallsalzbildender Verbindungen mit fluorhaltigen 1,3-Diketonen in direkter Reaktion und ohne Verwendung eines Lösungsmittels wasserfreie, fluorhaltige 1,3-Diketonato-Metall-Komplexe in hoher Ausbeute hergestellt werden, die sich als Chemikalien für die Oberflächenbehandlung von Glasmaterialien bzw. als Verschiebungsreagenzien für die Kernresonanzspektroskopie eignen.

EP 0 116 169 A2

Croydon Printing Company Ltd.

/

Merck Patentgesellschaft
mit beschränkter Haftung
D a r m s t a d t

Verfahren zur Herstellung wasserfreier, fluorhaltiger 1,3-Diketonato-Metall-Komplexe

Die Erfindung betrifft ein neues Verfahren für die Herstellung und die Verwendung wasserfreier, fluorhaltiger 1,3-Diketonato-Metall-Komplexe der allgemeinen Formel I

(I) 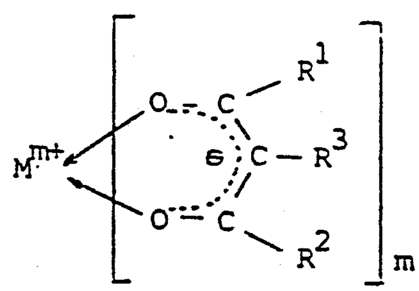

in dem

$M^{m+}$ = ein Metallkation der Wertigkeit m und
m = eine ganze Zahl von 1 bis 5,
wobei m und $m^+$ im Betrag immer gleich sind,
$R^1$ = Perfluoralkyl mit 1 bis 6 Kohlenstoffatomen,
$R^2$ = $R^1$, Alkyl, Cycloalkyl oder Aryl mit je bis zu 8 Kohlenstoffatomen und
$R^3$ = H oder $R^2$ ist,
wobei $R^2$ und $R^3$ auch gemeinsam Teil eines Cycloalkansystems sein können.

GSPHA18 B-fi

Komplexe dieser Art waren bisher nur sehr schwer zugänglich.

Metallkomplexe von nichtfluorierten 1,3-Diketonen sind schon länger literaturbekannt. Diese lassen sich nach konventionellen Methoden durch Umsetzung von 1,3-Diketonen mit Metallsalzen in wäßrigen Lösungen in Gegenwart von Basen erhalten (W.C. Fernelius, B.E. Bryant, Inorganic Synthesis 5, 105 (1957)).

Mit fluorhaltigen 1,3-Diketonen ist dieser Weg nicht immer möglich oder vorteilhaft. Meist ist das entsprechende fluorierte ß-Ketoenol in Wasser nicht beständig oder es entstehen Komplexhydrate, die gar nicht oder nur sehr schwer und unvollständig entwässert werden können. In einigen Fällen sind wasserfreie Synthesen ausgehend von dehydratisierten Metallhalogeniden in organischen Lösungsmitteln (M.L. Morris, R.W. Moshier und R.E. Sievers, Inorg. Chem. 2, 441 (1963)) oder ausgehend von Metallcarbonylen bekannt (T.G. Dunne, F.A. Cotton, Inorg. Chem. 2, 263 (1963)). Diese haben jedoch den Nachteil, daß absolute Lösungsmittel oder teure Ausgangsverbindungen verwendet werden müssen. Die direkte Einwirkung fluorierter 1,3-Diketone auf reine Metalle hat den Nachteil, daß lange Reaktionszeiten und unbefriedigende Ausbeuten in Kauf genommen werden müssen (R.E. Sievers, J.W. Connolly und W.D. Ross, J. Gas Chromatography 5, 241 (1967)).

Aufgabe der Erfindung war es nun, ein neues, allgemein anwendbares Herstellungsverfahren zu erarbeiten, durch das wasserfreie, fluorhaltige 1,3-Diketonato-Metall-Komplexe in einfacher Weise und mit hoher Ausbeute zugänglich gemacht werden können.

Diese Aufgabe wurde erfindungsgemäß gelöst dadurch, daß man ein wasserfreies Metallderivat $M^{m+}(X^-)_m$, in dem $X^-$ für den anionischen Rest einer flüchtigen, metallsalzbildenden Verbindung steht und m die Zahlen 1, 2, 3, 4, 5 bedeutet, mit einem fluorhaltigen 1,3-Diketon der allgemeinen Formel II

$$\text{(II)} \qquad R^1 - CO - CHR^3 - CO - R^2$$

worin $R^1$, $R^2$ und $R^3$ die oben angegebenen Bedeutungen haben, in direkter Reaktion ohne Lösungsmittel unter Abspaltung der flüchtigen Verbindung umsetzt.

Gegenstand der Erfindung ist somit dieses Verfahren zur Herstellung wasserfreier, fluorhaltiger 1,3-Diketonato-Metall-Komplexe, sowie die Verwendung der so hergestellten wasserfreien, fluorhaltigen 1,3-Diketonato-Metall-Komplexen zur Oberflächenbehandlung von Glasmaterialien sowie, vorzugsweise wenn es sich um Komplexe der Metalle der Lanthanidenreihe handelt, als Verschiebungsreagenzien für die Kernresonanzspektroskopie.

Die Herstellung von wasserfreien, fluorhaltigen 1,3-Diketonato-Metall-Komplexen nach dem erfindungsgemäßen Verfahren ist praktisch für alle Metalle des Periodensystems der Elemente möglich. Bevorzugt sind Komplexe von Metallen der Gruppe der Erdalkali- und Erdmetalle sowie der Lanthanidenreihe, wie z. B. Beryllium, Magnesium, Aluminium, Europium, Gadolinium. Aber auch Komplexe anderer Metalle, wie Titan, Zirkon, Zink können von Interesse sein.

Für das erfindungsgemäße Verfahren sind alle Metallderivate flüchtiger, metallsalzbildender Verbindungen geeignet.
Bevorzugt sind solche, die leicht zugänglich und wasserfrei erhältlich sind. Als flüchtige metallsalzbildende Verbindungen sind solche zu verstehen, die sich bei Temperaturen bis etwa 250 °C verdampfen und/oder zersetzen lassen. Als anionische Reste in den Metallderivaten kommen somit in Frage die Reste leichtverflüchtigbarer anorganischer Säuren, wie z. B. alle Halogenide, Nitrit, Nitrat, Sulfit bzw. Hydrogensulfit, Carbonat bzw. Hydrogencarbonat und Cyanid, die Reste leichtverflüchtigbarer organischer, metallsalzbildender Verbindungen, wie Carboxylat oder Alkoholat oder auch Hydrid. Bevorzugt sind Chlorid, Bromid, Jodid, Acetat und Ethylat.

Die nach dem erfindungsgemäßen Verfahren umzusetzenden 1,3-Diketone sind Verbindungen der allgemeinen Formel II, in denen $R^1$ = Perfluoralkyl mit 1 bis 6 C-Atomen $R^2$ = $R^1$, Alkyl, Cycloalkyl oder Aryl mit je bis zu 8 C-Atomen und $R^3$ = H oder $R^2$, wobei $R^2$ und $R^3$ auch

gemeinsam Teil eines Cycloalkansystems sein können. Sie können z.B. zusammen ein Cyclohexan- oder ein Campherringsystem bilden. Bevorzugt sind solche Verbindungen, in denen $R^1$ = niederes Perfluoralkyl mit bis zu 3 C-Atomen, $R^2$ = $R^1$ oder niederes Alkyl bzw. Isoalkyl mit bis zu 4 C-Atomen und $R^3$ = H ist oder $R^2$ und $R^3$ zusammen mit den sie verbindenen C-Atomen einen Campherringsystem bilden. Dies können beispielsweise sein die Verbindungen 1,1,1-Trifluoro-2,4-pentandion, 1,1,1,5,5,5-Hexafluoro-2,4-pentandion, 1,1,1,2,2,3,3-Heptafluoro-7,7-dimethyl-4,6-octandion oder 3-(2,2,2-Trifluoro-1-hydroxyethyliden)-d-campher.

Das erfindungsgemäße Verfahren zur Herstellung wasserfreier, fluorhaltiger 1,3-Diketonato-Metall-Komplexe wird so durchgeführt, daß man unter Ausschluß von Luftfeuchtigkeit, vorzugsweise durch Arbeiten unter Inertgas, die Reaktionspartner Metall-Derivat und 1,3-Diketon unter Rühren direkt und ohne noch ein Lösungsmittel zu verwenden, zusammenbringt, wobei die Reaktion meist sofort und unter Abspaltung der flüchtigen, metallsalzbildenden Verbindung in Gas- bzw. Dampfform einsetzt. Bei heftig einsetzender Reaktion kann es zunächst notwendig sein, die Reaktion durch Kühlung unter Kontrolle zu halten. Meist wird aber die Reaktion durch eine mäßige Erwärmung bis etwa zum Siedepunkt des 1,3-Diketons fortgesetzt, wobei sich im Verlaufe des Reaktionsfortschritts das Reaktionsgut verfestigt. Vorteilhaft ist es, zur Vervollständigung der Reaktion das Reaktionsgut weiter bis zum Schmelzen zu erwärmen und so lange auf Temperaturen bis maximal 250 °C zu halten, bis keine Gasentwicklung mehr erfolgt. Nach beendigter Reaktion wird das Produkt in der Regel durch Sublimation im Vakuum gereinigt.

Eine Anwendung dieser Verbindungen ist überall dort gegeben, wo besonders flüchtige Metallderivate benötigt werden und die Flüchtigkeit fluorhaltiger 1,3-Diketonato-Metall-Komplexe gegenüber den entsprechenden nichtfluorierten Komplexen Vorteile bringt und/oder wo auf den niedrigen Wasserstoffgehalt diese Verbindungen wertgelegt wird und Wasserfreiheit dieser Verbindungen unabdingbar ist.
Dies ist zum Beispiel der Fall bei der Verwendung solcher Verbindungen als Chemikalien bei der Oberflächenbehandlung von Glasmaterialien oder, insbesondere wenn es sich um Metalle der Lanthanidenreihe handelt, bei der Verwendung als Verschiebungsreagenzien für die Kernresonanzspektroskopie.

Nach dem erfindungsgemäßen Verfahren hergestellte wasserfreie, fluorhaltige 1,3-Diketonato-Metall-Komplexe, insbesondere der Erdalkali- und Erdmetalle, lassen sich in bekannter Weise sehr vorteilhaft bei der Oberflächenbehandlung von Glasmaterialien nach dem Chemical-Vapour-Deposition-Verfahren einsetzen. Hierbei werden diese Verbindungen verdampft und an heißen Oberflächen der zu behandelnden Glasmaterialien zersetzt, wobei durch das dabei abgeschiedene Metalloxid die Oberflächenzusammensetzung des Glases modifiziert wird. Vorteilhaft hierfür ist die leichtere Verdampfbarkeit aufgrund der höheren Flüchtigkeit der fluorhaltigen Komplexe gegenüber den entsprechenden nichtfluorierten Verbindungen, sowie der niedrigere Wasserstoffgehalt dieser Verbindungen.

Verbindungen für den hier beschriebenen Verwendungszweck sind nun nach dem erfindungsgemäßen Verfahren in einfacher Weise und mit hohen Ausbeuten zugänglich.

Aufgrund ihrer Wasserfreiheit und des geringen Gehaltes an Wasserstoff eignen sich nach dem erfindungsgemäßen Verfahren hergestellte fluorhaltige 1,3-Diketonato-Metall-Komplexe, wenn sie über ein paramagnetisches Moment verfügen, in besonderem Maße als Verschiebungsreagenzien für die Kernresonanzspektroskopie. Vorzugsweise werden hierfür Komplexe der Lanthanidenmetalle eingesetzt.
Der besondere Vorteil des erfindungsgemäßen Verfahrens ist neben der einfachen Herstellung darin zu sehen, daß die so hergestellten Verbindungen nicht mehr durch z. B. eine aufwendige Hochvakuumtrocknung entwässert werden müssen.

Die erfindungsgemäß hergestellten Substanzen werden in literaturbekannter Weise nach den bekannten Verfahren bei der Oberflächenbehandlung von Glasmaterialien bzw. als Verschiebungsreagenzien für die Kernresonanzspektroskopie verwendet.

Beispiel 1

Unter Stickstoff werden 73,1 g wasserfreies Berylliumdichlorid auf 60 °C erhitzt und unter Rühren 386 g Hexafluoro-2,4-pentandion langsam zugetropft, wobei Entwicklung von HCl-Gas auftritt. Nach Ende des Zutropfens wird auf 80 °C erwärmt und die Schmelze so lange bei dieser Temperatur gehalten, bis keine Gasentwicklung mehr zu beobachten ist. Nach dem Abkühlen wird das Rohprodukt bei 50 - 60 °C und 200 Pa sublimiert, wobei man 355 g Bis-(1,1,1,5,5,5-Hexafluoro-2,4-pentandionato)-beryllium erhält.

$^1$H-NMR (in $CDCl_3$): $\delta$ = 6,62 (s) ppm
Massenspektrum: m/e = 423

Beispiel 2

Unter Stickstoff werden 3,3 g wasserfreies Aluminiumtrichlorid bei Raumtemperatur tropfenweise mit 15,7 g Hexafluoro-2,4-pentandion versetzt. Man kühlt den Kolben von außen, um den Inhalt auf Raumtemperatur zu halten. Nach Beendigung des Zutropfens wird langsam auf 70 °C erhitzt, wobei das in der Zwischenzeit fest gewordene Reaktionsgemisch wieder schmilzt. Es wird noch 1 h bei dieser Temperatur nachgerührt. Dann läßt man unter Stickstoff abkühlen und sublimiert bei 55 °C und 200 Pa, wobei 12,3 g Tris-(1,1,1,5,5,5-hexafluoro-2,4-pentandionato)-aluminium erhalten werden.

$^1$H-NMR (in TDF): δ = 6,84 (s) ppm
Massenspektum: m/e = 648

Beispiel 3

Unter Stickstoff werden 10,4 g Magnesiumethylat bei Raumtemperatur mit 37,8 g Hexafluoro-2,4-pentandion versetzt. Man erwärmt langsam unter Rühren, wobei ein dicker Brei entsteht, der bei 70 °C fest wird. Weiteres Erhitzen führt bei 130 °C zum Schmelzen des Reaktionsgemisches. Man rührt noch 10 Minuten bei dieser Temperatur und läßt dann abkühlen. Nach Sublimation bei 90 °C und 200 Pa erhält man 27,3 g Bis-(1,1,1,5,5,5-hexafluoro-2,4-pentandionato)-magnesium.

$^1$H-NMR (in DMSO): δ = 5,6 (s) ppm
Massenspektrum: m/e = 438

Beispiel 4

5,2 g wasserfreies Zinkacetat werden unter Stickstoff bei Raumtemperatur mit 10 g Hexafluoro-2,4-pentandion versetzt. Man erwärmt langsam unter Rühren, wobei ein dicker Brei entsteht, der bei 70 °C fest wird. Weiteres Erhitzen führt bei 110 - 120 °C zum Schmelzen des Reaktionsgemisches. Man rührt noch 10 Min. bei dieser Temperatur und läßt dann abkühlen. Durch Sublimation bei 140 °C und 200 Pa erhält man 4,3 g Bis-(1,1,1,5,5,5-hexafluoro-2,4-pentandionato)-zink.

$^1$H-NMR (in DMSO): $\delta$ = 5,67 (s) ppm
Massenspektrum: m/e = 478

Beispiel 5

4,4 g wasserfreies Magnesiumchlorid werden unter Stickstoff bei Raumtemperatur mit 19,2 g Hexafluoro-2,4-pentandion versetzt. Man erwärmt langsam unter Rühren, wobei ein dicker Brei entsteht, der bei 60 °C fest wird. Weiteres Erhitzen führt bei 160 °C zum Schmelzen des Reaktionsgemisches. Man rührt noch so lange bis die Gasentwicklung aufhört und läßt dann abkühlen. Es wird bei 80 - 130 °C und 200 Pa im Vakuum sublimiert, wobei man 15,6 g Bis-(1,1,1,5,5,5-hexafluoro-2,4-pentandionato)-magnesium erhält.

Beispiel 6

5,2 g Zirkoniumtetrachlorid werden unter Stickstoff bei 0 °C mit 18,4 g Hexafluoro-2,4-pentandion versetzt. Nach Beendigung der Gasentwicklung wird auf

60 °C erhitzt und noch 10 Min. bei dieser Temperatur gerührt. Nach dem Abkühlen erhält man durch Sublimation bei 100 °C und 200 Pa 14,9 g Tetrakis-(1,1,1,5,5,5-hexafluoro-2,4-pentandionato)-zirkonium.

Massenspektrum: m/e = 505

Beispiel 7

2,8 g Gadoliniumtrichlorid werden unter Stickstoff bei Raumtemperatur mit 6,6 g Hexafluoro-2,4-pentandion versetzt. Man erwärmt langsam unter Rühren auf 70 °C. Nach Beendigung der Gasentwicklung läßt man abkühlen und sublimiert bei 120 °C und 200 Pa, wobei man 4,6 g Tris-(1,1,1,5,5,5-hexafluoro-2,4-pentandionato)-gadolinium erhält.

Massenspektrum: m/e = 571.

Beispiel 8

2,6 g Europiumtrichlorid werden unter Stickstoff bei Raumtemperatur mit 7,5 g 3-(2,2,2-Trifluoro-1-hydroxyethyliden)-d-campher versetzt. Man erwärmt langsam unter Rühren auf 80 °C. Nach Beendigung der Gasentwicklung läßt man abkühlen und sublimiert bei 130°C und 200 Pa, wobei man 5,7 g Tris-[3-(2,2,2-trifluoro-1-hydroxyethyliden)-d-camphorato]-europium erhält.

Massenspektrum: m/e = 893

Merck Patentgesellschaft
mit beschränkter Haftung
D a r m s t a d t

Patentansprüche

1. Verfahren zur Herstellung wasserfreier, fluorhaltiger 1,3-Diketonato-Metall-Komplexe der allgemeinen Formel (I)

(I) 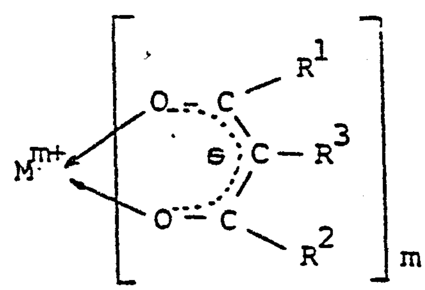

im dem

$M^{m+}$ = ein Metallkation der Wertigkeit m und
m = eine ganze Zahl von 1 bis 5,
wobei m und $m^+$ im Betrag immer gleich sind,
$R^1$ = Perfluoralkyl mit 1 bis 6 Kohlenstoffatomen,
$R^2$ = $R^1$, Alkyl, Cycloalkyl oder Aryl mit je bis zu 8 Kohlenstoffatomen und
$R^3$ = H oder $R^2$ ist,
wobei $R^2$ und $R^3$ auch gemeinsam Teil eines Cycloalkansystems sein können,

dadurch gekennzeichnet, daß man ein wasserfreies Metallderivat $M^{m+}(X^-)_m$, in dem

$X^-$ für den anionischen Rest einer flüchtigen, metallsalzbildenden Verbindung steht, mit einem fluorhaltigen 1,3-Diketon der allgemeinen Formel II

(II) $R^1 - CO - CHR^3 - CO - R^2$

worin $R^1$, $R^2$ und $R^3$ die oben angegebenen Bedeutungen haben, in direkter Reaktion ohne Lösungsmittel unter Abspaltung der flüchtigen Verbindung umsetzt.

2. Verfahren nach Anspruch 1, dadurch gekennzeichnet, daß man die Umsetzung unter Ausschluß von Luftfeuchtigkeit, vorzugsweise durch Arbeiten unter Inertgas, durchführt.

3. Verwendung von nach Verfahren gemäß den Ansprüchen 1 und 2 hergestellten wasserfreien, fluorhaltigen 1,3-Diketonato-Metall-Komplexen zur Oberflächenbehandlung von Glasmaterialien.

4. Verwendung von nach Verfahren gemäß den Ansprüchen 1 und 2 hergestellten wasserfreien, fluorhaltigen 1,3-Diketonato-Metall-Komplexen, vorzugsweise der Metalle der Lanthanidenreihe, als Verschiebungsreagenzien für die Kernresonanzspektroskopie.